(19) Europäisches Patentamt
European Patent Office
Office européen des brevets

(11) Numéro de publication: 0 014 295
B1

(12) # FASCICULE DE BREVET EUROPEEN

(45) Date de publication du fascicule du brevet: 19.01.83

(21) Numéro de dépôt: 79400071.1

(22) Date de dépôt: 05.02.79

(51) Int. Cl.³: C 07 D 405/06, A 61 K 31/335, A 61 K 31/445

(54) **Nouveaux dérivés du benzodioxanne, leur procédé de préparation et leurs applications en thérapeutique.**

(43) Date de publication de la demande:
20.08.80 Bulletin 80/17

(45) Mention de la délivrance du brevet:
19.01.83 Bulletin 83/3

(84) Etats contractants désignés:
BE CH DE FR GB IT LU NL SE

(56) Documents cités:
FR - A - 2 315 272
US - A - 2 056 046
US - A - 4 129 655

Le dossier contient des informations techniques présentées postérieurement au dépôt de la demande et ne figurant pas dans le présent fascicule.

(73) Titulaire: Bouchara, Emile
75 bis, Avenue Foch
F-75116 Paris (FR)

(72) Inventeur: Dumaitre, Bernard
47 Rue de Bretagne
F-75003 Paris (FR)
Inventeur: Perrin, Claude
47 Rue de Bretagne
F-75003 Paris (FR)
Inventeur: Cornu, Pierre-Jean
47 Rue de Bretagne
F-75003 Paris (FR)
Inventeur: Streichenberger, Gilles
47 Rue de Bretagne
F-75003 Paris (FR)

(74) Mandataire: Moncheny, Michel et al,
c/o Cabinet Lavoix 2 Place d'Estienne d'Orves
F-75441 Paris Cedex 09 (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 014 295 B1

Nouveaux dérivés du benzodioxanne, leur procédé de préparation et leurs applications en thérapeutique

La présente invention concerne de nouveaux dérivés du benzodioxanne, leur procédé de préparation et leurs applications en thérapeutique, notamment comme agents anti-hypertenseurs.

Les alcoylamino et les pipéridino-benzodioxannes sont des substances pharmacologiques dont les propriétés adrénolytiques ou sympatholytiques ont fait l'objet d'études depuis 1933. Ces travaux ont permis de mettre en évidence une dualité d'effets qui s'ajoutent ou se contrarient. Il en résulte que les benzodioxannes ont un effet imédiat, mais fugace, entraînant une réaction réflexe antagoniste. Ces substances sont donc à la fois hypotensives, puis hypertensives.

Par ailleurs, de nombreux travaux effectués depuis 1958 ont montré le grand intérêt présenté en thérapeutique par des pipéridines substituées qui présentent, en général, une action analgésique centrale du type morphinique (Palfium, Tilidine, Mépéridine, Fentanyl). Cette action analgésique est accompagnée souvent d'une action hypotensive, d'une action dépressive sur le système nerveux central et d'une action dépressive sur le rythme respiratoire.

C'est pourquoi les chercheurs se sont efforcés de trouver des pipéridines présentant un effet pur soit dépresseur du système nerveux central (butyrophénones), soit antalgique (Fentomyl), soit encore antihypertensif.

Une première approche dans la recherche d'un effet antihypertensif a été la préparation de l'Indoramine et des analogues de structure qui possèdent principalement des propriétés $\alpha$-adrénolytiques ainsi que des propriétés anti-histaminiques et des propriétés sédatives.

Ultérieurement, Ciba-Geigy a préparé des benzodioxanly pipéridines substituées par un groupe aralcoyle ou aryle en position 4. Ces composés ont des propriétés neuroleptiques et tranquilissantes.

Il a maintenant été découvert qu'en substituant la position 4 du noyau pipéridique par un substituant benzoyle les propriétés pharmacologiques étaient complètement différentes et même surprenantes. Les propriétés antihypertensives des composés selon l'invention sont différentes de celles du Pipéroxan, comme de celles des autres pipéridines, en ce qu'elles sont accompagnées d'un minimum d'effets secondaires liés à un effet sur le système nerveux central.

Ces propriétés sont surprenantes en ce que leur durée d'action est indépendante de leur intensité d'action.

Il en résulte donc que les composés selon l'invention sont de véritables médicaments de l'hypertension, manifestant une efficacité certaine et durable, ne s'atténuant pas avec la répétition des doses et ne se potentialisant pas au cours d'un traitement prolongé.

Le retour à la pression initiale se fait progressivement sans phase d'hypertension.

La présente invention a pour objet des composés de formule

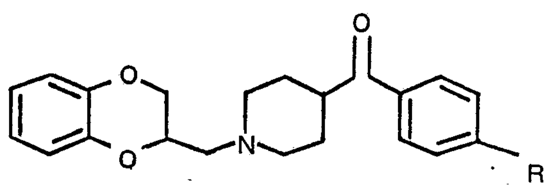

(I)

dans laquelle R représente un atome d'hydrogène, un groupe alkyle en $C_1$ à $C_6$, un atome d'halogène, un groupe hydroxy, un groupe alcoxy en $C_1$ à $C_6$ ou un groupe acyloxy,
ainsi que leurs sels d'addition avec des acides pharmaceutiquement acceptables.

Le procédé général de préparation consiste à condenser des 4-benzoyl pipéridine substituées correspondantes, soit avec le 2-chlorométhyl-1,4-benzodioxanne, soit de préférence avec un ester réactif du 2-hydroxyméthyl-1,4 benzodioxanne tel que le méthanesulfonate ou le p-toluènesulfonate.

La fonction cétone des 4-benzoyl pipéridines peut être protégée en formant un dioxolanne par réaction avec l'éthylène-glycol en présence d'acide p-toluènesulfonique.

La fonction cétone est ensuite régénérée par hydrolyse acide.

Le schéma de la réaction est le suivant

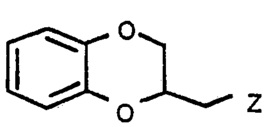

+

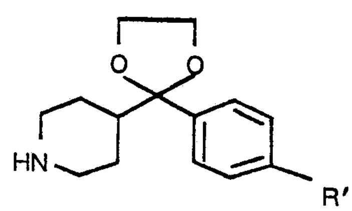

(II) (III)

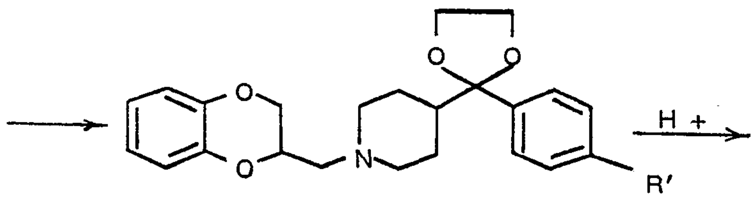


(IV)

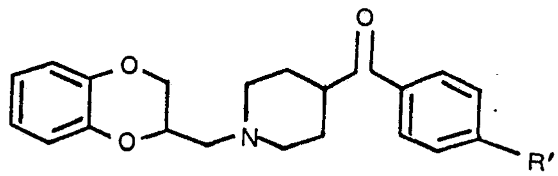

(I)

Dans ce schéma, R′ désigne un atome d'hydrogène, un atome d'halogène, un groupe alkyle ou un groupe alcoxy et Z représente un atome de chlore ou un reste d'ester réactif.

La réaction de condensation est conduite dans un solvant convenable, tel que le xylène à l'ébullition, ou le diméthylformamide, en présence d'un accepteur d'acide tel que le carbonate de potassium.

Le composé dans lequel R = OH peut être obtenu à partir des composés dans lesquels R est un groupe alcoxy par action d'acide bromhydrique.

Les composés dans lesquels R est un groupe acyloxy peuvent être obtenus par acylation du composé hydroxylé.

Les produits de départ sont préparés par des procédés classiques. Ainsi, le méthane sulfonate et le paratoluène sulfonate du 2-hydroxyméthyl-2,3-dihydro-4H-1,4-benzodioxine, peuvent être obtenus par condensation de l'épichlorhydrine sur le pyrocatéchol en milieu alcalin et traitement de la 2-hydroxyméthyl-2,3-dihydro-4H-1,4-benzodioxine obtenue par le chlorure de méthanesulfonyle ou le chlorure de p-toluènesulfonyle.

Les benzoylpipéridines nécessaires sont obtenues par réaction de Friedel et Crafts du chlorhydrate du chlorure de l'acide N-acétylisonipécotique sur les benzènes substitués adéquats suivie de désacétylation par ébullition en milieu chlorhydrique. On peut aussi utiliser la réaction de Friedel et Crafts du chlorhydrate du chlorure de l'acide 4-pyridine-carboxylique sur les benzènes substitués adéquats, suivie de l'hydrogénation catalytique des produits obtenus.

Les exemples ci-dessous illustrent l'invention.

Dans ces exemples, la nomenclature utilisée est celle des dernières années des Chemical Abstracts.

En outre, toutes les parties sont en poids, sauf indication contraire.

## Exemple 1

a) 4-[(4-Fluoro-$\alpha$-éthylènedioxy)benzyl]-piperidine

Dans un réacteur muni d'un séparateur d'eau du type Dean-Stark, on porte 16 heures à l'ébullition, sous agitation, 22 parties de 4-(4-fluorobenzoyl)pipéridine, 21 parties d'acide p-toluènesulfonique, 50 parties d'ethylèneglycol et 250 parties de benzène, tout en éliminant l'eau par azéotropie. Après refroidissement à 0°C, on ajoute 65 parties de soude 2N et sépare la phase organique par décantation. Cette dernière est lavée à l'eau, séchée et concentrée sous préssion réduite.

On obtient ainsi 21 parties d'un solide blanc.

Fi = 70°C.

Le produit est assez pur pour être utilisé dans la suite de la synthèse.

b) Chlorhydrate de la 1-[(2,3-dihydro-4H-1,4-benzodioxin)-2-ylméthyl]-4-[(4-fluoro-$\alpha$-éthylènedioxy)-benzyl] pipéridine

On porte 18 heures à l'ébullition avec agitation, un mélange de 27 parties de méthanesulfonate de la 2-hydroxyméthyl-2,3-dihydro-4H-1,4-benzodioxine, 25 parties de 4-[(4-fluoro-$\alpha$-éthylènedioxy)-benzyl] pipéridine, 15 parties de carbonate de potassium et 200 parties de toluène. Après refroidissement, on filtre et acidifie le filtrat sous forte agitation par 50 parties d'HCl à 20 pour cent.

Les cristaux formés sont essorés et lavés à l'éther.

On obtient ainsi 26 parties de produit de

Fi = 220°C.

c) 1-[(2,3-dihydro-4H-1,4-benzodioxin)-2-ylméthyl]-4-(4-fluorobenzoyl)pipéridine

On porte pendant 2 h. 30 mn à l'ébullition un mélange de 100 parties du chlorhydrate de 1-[(2,3-dihydro-4H-1,4-benzodioxin)-2-ylméthyl]-4-[(4-fluoro-$\alpha$-éthylènedioxy)benzyl] pipéridine, 200 parties d'HCl concentré, 400 parties d'isopropanol et 100 parties d'eau.

On concentre ensuite à sec, traite à l'eau, basifie légèrement par NaOH, extrait à l'acétate d'éthyle et sépare la phase organique.

Après lavage à l'eau, séchage et concentration à sec, on obtient 74 parties d'un solide blanc.

Fi: 95°C—96°C.

Exemple 2

Chlorhydrate de la 1-[(2,3-dihydro-4H-1,4-benzodioxin)-2-ylméthyl]-4-(4-fluorobenzoyl) pipéridine

On dissout 50 parties de 1-[(2,3-dihydro-4H-1,4-benzodioxin)-2-ylméthyl]-4-(4-fluorobenzoyl) pipéridine dans 1000 parties d'éther et ajoute la quantité stoechiométrique de solution d'acide chlorhydrique dans l'éther anhydre, ce qui provoque la précipitation du chlorhydrate.

Après filtration et lavage à l'éther, on obtient 52 parties de produit qui peut être purifié par recristallisation dans l'alcool isopropylique.

On obtient des écailles blanches.

Fi: 195—196°C.

Exemple 3

1-[(2,3-Dihydro-4H-1,4-benzodioxin)-2-ylméthyl]-4-(4-méthoxy-benzoyl) pipéridine.

On porte 16 heures à l'ébullition, sous agitation, 24 parties de méthanesulfonate de 2-hydroxyméthyl-2,3-dihydro-4H-1,4-benzodioxine, 22 parties de 4-méthoxybenzoylpipéridine, 14 parties de carbonate de potassium dans 200 parties de xylène anhydre.

Après refroidissement, on lave à l'eau et traite par l'acide chlorhydrique normal en phase hétérogène, ce qui provoque la précipitation du chlorhydrate du produit cherché sous forme d'huile.

Cette huile est séparée et la base est libérée par la soude diluée et extraite à l'acétate d'éthyle.

Après lavage à l'eau, séchage et concentration, on obtient 25 parties de produit qui reste sous forme d'huile.

Exemple 4

Fumarate de la 1-[(2,3-dihydro-4H-1,4-benzodioxin)-2-ylméthyl]-4-(4-méthoxybenzoyl) pipéridine

25 parties de la 1-[(2,3-dihydro-4H-1,4-benzodioxin)-2-ylméthyl]-4-(4-méthoxybenzoyl) pipéridine sont dissoutes dans 100 parties d'éthanol et l'on ajoute 8 parties d'acide fumarique en solution dans 100 parties d'éthanol.

On filtre et laisse cristalliser ce qui conduit à 19 parties de fumarate que l'on peut purifier par recristallisation dans l'éthanol.

Cristaux blancs.

Fi = 170°C.

Exemple 5

1-[(2,3-Dihydro-4H-1,4-benzodioxin)-2-ylméthyl]-4-(4-chloro-benzoyl) pipéridine

On porte 16 heures à lébullition, sous agitation, un mélange de 12,5 parties de méthanesulfonate de 2-hydroxyméthyl-2,3-dihydro-4H-benzodioxine, 10,5 parties de 4-chlorobenzoylpipéridine et 7 paties de carbonate de potassium dans 200 parties de xylène anhydre.

Après refroidissement, on lave à l'eau et traite par l'acide chlorhydrique normal en phase hétérogène.

Après précipitation, l'huile obtenue est séparée et on libère la base par la soude diluée.

Après extraction à l'acétate d'éthyle, lavage à l'eau et concentration, on obtient un solide qui est recristallisé dans l'éther isopropylique avec traitement au noir animal.

On obtient 5 parties de produit cristallisé.

Fi = 120°C.

Exemple 6

Chlorhydrate de la 1-[(2,3-dihydro-4H-1,4-benzodioxine)-2-ylméthyl]-4-(4-chlorobenzoyl) pipéridine

5 parties de 1-[2,3-dihydro-4H-1,4-benzodioxin)-2-ylméthyl]-4-(4-chlorobenzoyl) pipéridine sont dissoutes dans 50 parties d'éthanol à chaud et ou ajoute la quantité stoechiométrique d'éther chlorhydrique.

Il y a cristallisation au refroidissement et après séparation, on obtient 4,3 parties de cristaux blancs.

Fi = 223°C.

Exemple 7

1-[(2,3-Dihydro-4H-1,4-benzodioxin)-2-ylméthyl]-4-(4-hydroxy-benzoyl) pipéridine

On porte 2 heures à l'ébullition 32 parties de 1-[(2,3-dihydro-4H-1,4-benzodioxin)-2-ylméthyl]-4-(4-méthoxy-benzoyl) pipéridine avec 400 parties d'acide bromhydrique à 48%.

Après refroidissement, on verse dans l'eau glacée et le solide qui précipite est séparé et lavé à l'eau.

Il est ensuite dissous à chaud dans le méthanol et basifié par $NH_4OH$.

La solution obtenue est concentrée à sec et extraite à l'acétate d'éthyle.

Après lavage, séchage et concentration, on obtient un solide qui est recristallisé dans le méthanol.

On récupère 22,5 parties de cristaux blancs.

Fi = 94,5°C.

Exemple 8

Nicotinate de la 1-[(2,3-dihydro-4H-1,4-benzodioxin)-2-ylméthyl]-4-(4-hydroxybenzoyl) pipéridine

On dissout 3,5 parties de 1-[(2,3-dihydro-4H-1,4-benzodioxin)-2-ylméthyl]-4-(4-hydroxybenzoyl) pipéridine dans 35 parties de benzène et 20 parties de pyridine.

On ajoute avec agitation 4 parties du chlorhydrate du chlorure de nicotinoyle par petites portions, puis on chauffe à douce ébullition pendant 16 heures.

On verse ensuite dans l'eau, extrait à l'acétate d'éthyle, lave la phase organique à la soude diluée, puis à l'eau et concentre.

On obtenient 3 parties de cristaux blancs que l'on peut purifier par recristallisation dans l'éthanol.

Fi = 125°C.

Exemple 9

Chlorhydrate de la 1-[(2,3-dihydro-4H-1,4-benzodioxin)-2-ylméthyl]-4-benzoyl pipéridine

Dans un réacteur muni d'une agitation, on porte 18 heures au reflux un mélange de 23,5 parties de 4-[$\alpha$-éthylène-dioxybenzyl] pipéridine, de 24,5 parties de méthanesulfonate de 2-hydroxyméthyl-2,3-dihydro-4H-1,4-benzodioxine, 14 parties de carbonate de potassium et 300 ml de toluène.

Après refroidissement, on filtre et sous agitation ajoute au filtrat 100 parties d'HCl à 20 pour cent.

Après filtration des cristaux formés, lavage à l'éther, on obtient 29,5 parties de chlorhydrate de la 1-[(2,3-dihdyro-4H-1,4-benzodioxin)-2-ylméthyl]-4-($\alpha$-éthylènedioxybenzyl) pipéridine.

Fi = 235°C.

29,5 parties de ce produit sont ensuite portées à l'ébullition avec 110 parties d'isopropanol, 30 parties d'$H_2O$ et 30 parties d'HCl concentré.

Après évaporation à sec, on traite à l'eau, neutralise par NaOH diluée et extrait à l'acétate d'éthyle pour obtenir des cristaux blancs qui sont recristallisés dans l'éther isopropylique pour fournir 21 parties de 1-[(2,3-dihydro-4H-1,4-benzodioxin)-2-ylméthyl]-4-benzoylpipéridine.

Fi = 141°C

Ce dernier est transformé en chlorhydrate par réaction avec HCl dans l'isopropanol.

Cristaux blancs. Fi = 190—192°C.

Exemple 10

(Chlorhydrate de la 1-[(2,3-dihydro-4H-1,4-benzodioxin)-2-ylméthyl]-4-(4-méthylbenzoyl) piperidine

Dans un réacteur muni d'une agitation, on porte 24 heures à 100°C un mélange de 25 parties de 4-(4-méthyl-$\alpha$-éthylène dioxybenzyl) pipéridine, de 24,5 parties de méthanesulfonate de 2-hydroxyméthyl-2,3-dihydro-4H-1,4-benzodioxine, 14 parties de carbonate de potassium et 300 parties de diméthylformamide.

Après refroidissement, on chasse le D.M.F. sous pression réduite, reprend à l'eau et extrait à l'éther.

On ajoute ensuite, sous forte agitation, à la phase éthérée lavée à l'eau, 100 parties d'HCl à 20%, ce qui provoque la formation de cristaux.

Ceux-ci sont filtrés, lavés à l'eau puis à l'éther et séchés, ce qui permet d'obtenir 21,5 parties de chlorhydrate de la 1-[(2,3-dihydro-4H-1,4-benzodioxin)-2-ylméthyl]-4-(4-méthyl-$\alpha$-éthylène dioxybenzyl) pipéridine.

Fi = 225°C.

21,5 parties de ce produit sont ensuite portées 2 h.30 mn à l'ébullition avec 120 parties d'isopropanol, 30 parties d'$H_2O$ et 60 parties d'HCl concentré.

Après évaporation à sec, le résidu est repris à l'eau, neutralisé par NaOH et extraite à l'acétate d'éthyle, ce qui permet d'obtenir 19 parties de cristaux blancs de 1-[(2,3-dihydro-4H-1,4-benzodioxin)-2-ylméthyl]-4-(4-méthylbenzoyl) pipéridine.

Fi = 114°C.

Ce dernier est transformé en chlorhydrate per réaction avec HCl dans l'isopropanol.

Cristaux blancs. Fi = 250°C.

Exemple 11

Chlorhydrate de la 1-[(2,3-dihydro-4H-1,4-benzodioxin)-2-ylméthyl]-4-(4-éthylbenzoyl) pipéridine

Dans un réacteur muni d'une agitation, on porte 24 heures à 100°C un mélange de 27 parties de

4-(4-éthyl-$\alpha$-éthylène-dioxybenzyl) pipéridine, de 24,5 parties de méthanesulfonate de 2-hydroxyméthyl-2,3-dihydro-4H-1,4-benzodioxine, 14 parties de carbonate de potassium et de 300 parties de diméthylformamide.

Après réaction, le D.M.F. est éliminé sous pression réduite, le résidu repris par l'eau et extrait à l'éther.

On ajoute ensuite, sous agitation, à la solution éthérée, 100 parties d'HCl à 20 pour cent, ce qui provoque la cristallisation du chlorhydrate de la 1-[(2,3-dihydro-4H-1,4-benzodioxin)-2-ylméthyl]-4-(4-éthyl-$\alpha$-éthylènedioxybenzyl) pipéridine.

On obtient 26 parties de produit.

Fi = 250°C.

22 parties de ce produit sont ensuite portées 2 h 30 mn à l'ébullition avec 120 parties d'isopropanol, 30 parties d'$H_2O$, 60 parties d'HCl concentré.

Après évaporation à sec, reprise par l'eau et neutralisation, on extrait à l'acétate d'éthyle, ce qui permet d'obtenir, après traitement habituel 19,3 parties de 1-[(2,3-dihydro-4H-1,4-benzodioxin)-2-ylméthyl]-4-(4-éthylbenzoyl) pipéridine, sous forme d'huile.

Cette huile est transformée en chlorhydrate par réaction avec HCl dans l'isopropanol.

Cristaux blancs. Fi = 205°C.

Les composés de formule I et leurs sels d'addition pharmaceutiquement acceptables possèdent des propriétés pharmacologiques intéressantes, en particulier dans le domaine cardiovasculaire. C'est ainsi qu'ils provoquent une diminution rapide et durable de la tension artérielle chez l'animal normotendu et chez l'animal hypertendu. Cet effet anti-hypertenseur s'accompagne dans certains cas d'une diminution de la fréquence cardiaque. Les premiers essais chez l'homme ont confirmé ces résultats. On a en outre observé chez l'animal une diminution du taux des triglycérides, ainsi que des effets au niveau du système nerveux central (effet neurodépresseur et analgésie). Leur toxicité n'apparaît qu'à des doses très supérieures aux doses pharmacologiques activies, ce qui permet leur utilisation en thérapeutique, notamment comme antihypertenseurs.

On donnera ci-après des résultats des essais pharmacologiques et toxicologiques. Ces résultats sont donnés principalement pour le composé de l'exemple 2 qui présente le meilleur indice thérapeutique.

I — *Propriétés cardiovasculaires*

a) Effet sur la pression artérielle de l'animal normotendu anesthésié.

L'étude a été conduite chez le rat, le lapin et le chien. Le composé de l'exemple 2 a été administré par voie intraveineuse. Les différents paramètres enregistrés ont été les pressions artérielles systoliques, diastoliques et moyennes, la fréquence cardiaque, la vitesse d'éjection systolique (dp/dt). La force de contraction cardiaque, les débits artériel et veineux ont été enregistrés chez le chien uniquement.

La composé de l'exemple 2 entraîne dès la dose de 0,250 mg/kg une chute tensionnelle systo-diastolique très rapide, proportionnelle aux doses tant en intensité qu'en durée, accompagnée de bradycardie chez le rat. Chez le chien, il a été observé, parallèlement à l'hypotension une augmentation de la force de contraction cardiaque, du dp/dt et des débits artériel et veineux.

Le composé de l'exemple 2 est donc dans ces conditions expérimentales un hypotenseur puissant n'entraînant pas de dépression cardiaque.

b) Effet sur la pression artérielle de l'animal hypertendu non anesthésié.

L'étude a été conduite chez le rat et chez le chien. On a utilisé, d'une part, des animaux (rats) présentant une hypertension expérimentale induite par ligature de l'aorte abdominale entre les deux artères rénales et, d'autre part, des animaux (rats et chiens) porteurs d'hypertension spontanée.

Le composé de l'exemple 2 a été administré par voie orale soit en prise unique pour connaître la cinétique d'action du produit, soit en traitement prolongé.

Sur des modèles expérimentaux, il présente une action antihypertensive nette, prolongée, augmentant avec les doses jusqu'à un plafond. Chez le chien, on peut noter une persistance de l'effat antihypertenseur après arrêt du traitement avec retour progressif à la normale.

Les tableux I et II donnent les résultats obtenus avec le composé de l'exemple 2 respectivement chez le rat et chez le chien.

TABLEAU I

| Dose mg/kg P.O. | Diminution de la pression artérielle en mm de mercure Chez le rat | | | | | | | |
|---|---|---|---|---|---|---|---|---|
| | 10mn | 30mn | 1h | 2h | 6h | 12h | 18h | 24h |
| 10 | 39±11 | 45±10 | 33±9 | 20±6 | 6±3 | — | — | — |
| 20 | 66±8 | 63±6 | 49±7 | 37±7 | 29±6 | 17±7 | 8±4 | 3±3 |
| 50 | 68±6 | 68±5 | 52±6 | 38±7 | 32±8 | 16±7 | 8±5 | 5±5 |

TABLEAU II

| Dose mg/kg P.O. | Diminution de la pression artérielle en mm de mercure Chez le chien | | | | | | |
|---|---|---|---|---|---|---|---|
| | 20mn | 40mn | 1h | 2h | 5h | 7h | 24h |
| 10 | 10±4 | 10±4 | 18±3 | 11±1 | 4±3 | 1±2 | 0±1 |
| 20 | 12±4 | 26±3 | 38±2 | 37±3 | 18±7 | 8±1 | 2±3 |
| 50 | 21±9 | 31±6 | 40±8 | 46±3 | 28±8 | 22±6 | 1±2 |

II — *Effets sur le système nerveux central*

Chez la souris, on a observé, lors de l'administration du composé de l'exemple 2 par voie orale, une sédation générale, une diminution de l'agressivité, une potentialisation du sommeil expérimental, qui apparaissent à des doses provoquant une activité antihypertensive chez le rat. Ces effets centraux sont peut-être directement liés à l'action hypotensive

III — *Toxicité*

a) Toxicité aiguë

La toxicité a été déterminée chez la souris par voie orale. Les résultats sont donnés dans le tableau III.

TABLEAU III

| Exemple | Dose léthale 50 approchée mg/kg per os |
|---|---|
| 2 | 880 |
| 4 | 364 |
| 6 | 880 |
| 7 | 440 |
| 8 | >3200 |
| 9 | 2400 |
| 10 | 1460 |
| 11 | 1200 |

b) Toxicité chronique

Le composé de l'exemple 2, mélangé à la nourriture, a été administré 45 jours de suite à des rats.

— A 75 mg/kg/jour, il n'entraîne aucune anomalie ni clinique, ni hématologique, ni anatomique. Au point de vue biochimique, on observe une chute nette et isolée du taux des triglycérides.

**Revendications**

1. Composés de formule

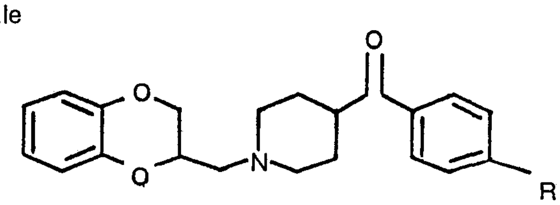

(I)

dans laquelle R représente un atome d'hydrogène, un atome d'halogène, un groupe alkyle en $C_1$ à $C_6$, un groupe hydroxy, un groupe alcoxy en $C_1$ à $C_6$ ou un groupe acyloxy,
et leurs sels d'addition avec des acides pharmaceutiquement acceptables.

2. Composé de formule I telle que définie à la revendication 1, dans laquelle R est un atome de fluor et ses sels d'addition avec des acides pharmaceutiquement acceptables.

3. Composition thérapeutique ayant notamment un effet anti-hypertenseur, caractérisée en ce qu'elle contient à titre de principe actif un composé selon la revendication 1 ou la revendication 2.

4. Procédé de préparation d'un composé de formule I telle que définie à la revendication 1, caractérisé en ce que l'on condense une 4-benzoyl-pipéridine de formule

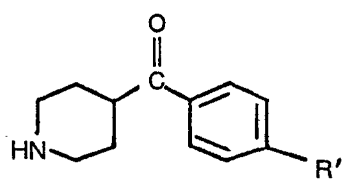

dans laquelle R′ désigne un atome d'hydrogène, un atome d'halogène, un groupe alkyle ou un groupe alcoxy,
avec un composé de formule

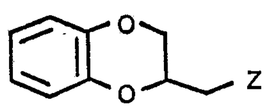

dans laquelle Z représente un atome de chlore ou un reste d'ester réactif,
et pour obtenir le composé dans lequel R est un groupe hydroxy on traite un composé dans lequel R est un groupe alcoxy par l'acide bromhydrique,
et pour obtenir les composés dans lesquels R est un groupe acyloxy on effectue une acylation du composé dans lequel R est un groupe hydroxy.

5. Procédé selon la revendication 4, caractérisé en ce qu'on protège la fonction cétonique de la 4-benzoyl pipéridine sous forme d'un dioxolanne.

**Claims**

1. Compounds having the formula:

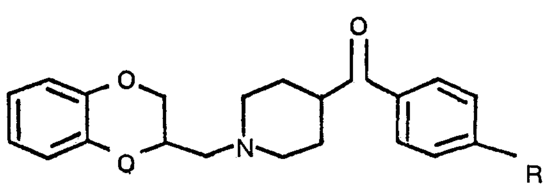

(I)

in which R is selected from hydrogen, halogen, $C_{1-6}$ alkyl, hydroxy, $C_{1-6}$ alkoxy and acyloxy,
and their pharmaceutically acceptable acid addition salts.

2. Compound of the formula (I) as defined in claim 1, wherein R is a fluorine atom, and its pharmaceutically acceptable acid-addition salts.

3. Therapeutic composition having, in particular, an anti-hypertensive effect, comprising, as active ingredient, a compound as claimed in claim 1 or claim 2.

4. Process for the preparation of a compound of the formula (I) as claimed in claim 1, comprising condensing a 4-benzoyl-piperidine having the formula:

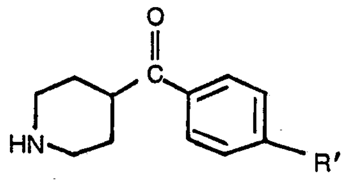

in which R' is selected from hydrogen, halogen, alkyl and alkoxy, with a compound having the formula:

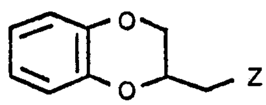

in which Z is selected from a chlorine atom and a reactive ester residue, and, to give the compound in which R is a hydroxy group, treating a compound in which R is alkoxy with hydrobromic acid,
and, to give the compounds in which R is acyloxy, acylating the compound in which R is hydroxy.

5. Process as claimed in claim 4, wherein the ketone function of 4-benzoyl piperidine is protected as the dioxolan.

**Patentansprüche**

1. Verbindungen der Formel

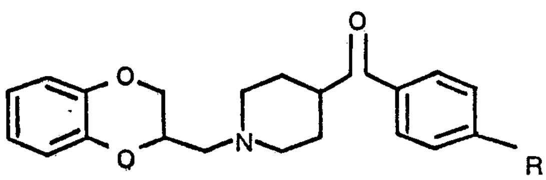

(I)

in der R ein Wasserstoffatom, ein Halogenatom, ein $C_1$—$C_6$-Alkylrest, eine Hydroxylgruppe, ein $C_1$—$C_6$-Alkoxyrest oder ein Acyloxyrest ist,
und ihre Additionssalze mit pharmazeutisch unbedenklichen Säuren.

2. Verbindung der Formel I wie in Anspruch 1 definiert, in der R ein Fluoratom ist, und ihre Additionssalze mit pharmazeutisch unbedenklichen Säuren.

3. Arzneimittelzubereitung mit insbesondere antihypertonischer Wirkung, dadurch gekennzeichnet, daß sie als Wirkstoff eine Verbindung nach Anspruch 1 oder Anspruch 2 enthält.

4. Verfahren zur Herstellung einer Verbindung der Formel I, wie sie in Anspruch 1 definiert ist, dadurch gekennzeichnet, daß man ein 4-Benzoylpiperidin der Formel

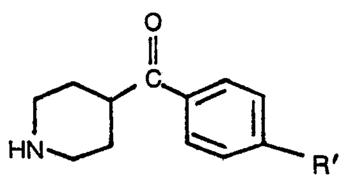

in der R' ein Wasserstoffatom, ein Halogenatom, ein Alkylrest oder ein Alkoxyrest ist, mit einer Verbindung der Formel

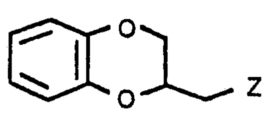

in der Z ein Chloratom oder ein reaktiver Esterrest ist, kondensiert
und zur Gewinnung der Verbindung, in der R eine Hydroxylgruppe ist, eine Verbindung, in der R ein Alkoxyrest ist, mit Bromwasserstoffsäure behandelt

und zur Gewinnung der Verbindungen, in denen R ein Acyloxyrest ist, eine Acylierung der Verbindung vornimmt, in der R eine Hydroxylgruppe ist.

5. Verfahren nach Anspruch 4, dadurch gekennzeichnet, daß man die Ketonfunktion des 4-Benzoylpiperidins in Form eines Dioxolans schützt.